# EUROPEAN PATENT APPLICATION

(11) **EP 0 689 855 A1**
(43) Date of publication of application: **03.01.1996**
(21) Application number: 94304334.9
(22) Date of filing: 15.06.1994
(51) Int. Cl.: A61N 1/36, A61H 39/00

(54) **Abdomen slimming apparatus**

(71) Applicant: WING HUNG TRADING COMPANY, Happy Valley (HK)
(72) Inventor: Yao, Chong De, Nan Men, Nan Kai District, Tianjin (CN); Wang, Xin Tang, Nan Men, Nan Kai District, Tianjin (CN)
(74) Representative: SERJEANTS

(57) **Abstract**

An abdomen slimming apparatus comprising a timing control circuit (30), a curing pulse generating circuit (20), a power amplifying and output circuit (40), a curing pulse signal intensity indicating circuit (50) and electrode contacts (10). The electrode contacts (10) are movable magnetic electrodes positioned in electrode holes in the bottom of the slimming apparatus, such that said slimming apparatus may be placed directly on the abdomen of a slimmer during treatment. The slimming effect is achieved by stimulating the acupuncture points with a combined short acupuncture pulse wave and long acupuncture pulse wave.

## Description

### Field of the Invention

The present invention relates to an electronic medical instrument for slimming the human body, and more particularly to an abdomen slimming apparatus utilizing electric pulses to stimulate the acupuncture points on the human body to achieve a slimming effect.

### Background of the Invention

It is well known that a corpulent body not only impairs the gracefulness of the human body but also causes many diseases that affect health, such as cardiac diseases, hypertension and apoplexy in worse cases as well as joint damage due to overload of the joints by the excessive weight of the body in less serious cases.

In order for a corpulent person to slim his or her body, he or she may diet and engage in a large amount of sport to achieve the object of slimming. However, such a slimming method needs perseverence and willpower, and a vigorous and active character as well as proceeding in an orderly way and step by step. With this method it is impossible to achieve the goal of slimming in a short period of time, and it will often result in failure by giving up halfway and wasting one's previous efforts for lack of perseverance and willpower.

Besides the above-mentioned methods of slimming, there are other methods emerging, for example, taking orally or applying externally slimming medicines, and undergoing an operation to extract fat. However, most slimming medicines have certain side-effects, and slimming using medicines generally inhibits the natural functions of the human body, so should be carried out strictly under the monitoring of doctors.

Operations for extracting fat for slimming, if improperly carried out, may result in dreadful consequences, for example, an improper operation for extraction of fat may result in pustules, bumps and holes, or inflammation on the area being extracted: it will be full of scars and look very ugly.

Nowadays, there are also electronic slimming apparatus developed by utilizing Chinese traditional medical theories combined with modern electronic technology, especially portable electronic slimming apparatus. Their electrode contacts are separated ones, and are attached to the curing acupuncture points in use, therefore the use of such apparatus requires some knowledge of Chinese traditional medicine and meridian theories, whereby there are difficulties in the popularization of the use, especially home use, of these apparatus. Some of the electronic slimming apparatus will cause uncomfortable side-effects, while the existing single function portable electronic slimming apparatus are of single wave curing pulses, whose curing effect is rather poor.

### Summary of the Invention

It is a primary object of the present invention to provide an efficient abdomen slimming apparatus which outputs combined waveform curing signals, so as to overcome the disadvantages and drawbacks of the above-mentioned slimming methods.

It is another object of the present invention to provide an abdomen slimming apparatus which is miniature in size, simple in construction, easy to carry and use, and adaptive to be operated and used at home and by persons without the supervision of medical personnel.

It is yet another object of the present invention to provide an abdomen slimming apparatus which employs fewer acupuncture points and is easy to align on these acupuncture points.

The above tasks are implemented as follows: said abdomen slimming apparatus is developed based on the theories of organs and visceras, meridian, acupuncture and moxibustion of Chinese traditional medicine and incorporated modern electronic technology. The slimming apparatus generates electrical pulses to replace the needles and acupuncture effects of acupuncture, the pulse waveforms include high frequency stimulation pulse waves for short acupuncture (hereinafter referred as short acupuncture pulse waves) and strong stimulation pulse waves for lifting and inserting quiver (hereinafter referred as long acupuncture pulse waves). The slimming apparatus combines the short acupuncture pulse waves and the long acupuncture pulse waves thus generated and then outputs the combined pulse waves, which are applied on the abdominal slimming curing acupuncture points via the electrode contacts and stimulate the acupuncture points with electric pulses, which are similar to the stimulation on the acupuncture points by the acupuncture and twirling of the needle of normal Chinese traditional acupuncture. However, the frequency of acupuncture and intensity of stimulation are much stronger than that of ordinary acupuncture.

The result of slimming experiments on animals (healty adult white rabbits) of the abdomen slimming apparatus according to the present invention has proven the following slimming mechanism: retraction of the subcutaneous tissues and muscles occurs by pulsed electric stimulation of the abdominal acupuncture points of the rabbit's abdomen. Energy consumption is required during continuous exercises, and the energy comes from the nearest energy supply, i.e. the adjacent adipose tissue, which releases energy through the biochemical conversion of glycerin tripulmitate to accommodate the energy consumption for musclar retraction. At the same time, the fat drop in the adipose cell is retracted due to the energy supply therefrom, and the volume of the cell is reduced. Thus, the effect of slimming is attained. The slimming and abdominal retraction by the pulsed electric stimulation of the apparatus according to the present invention has been proved by the above-mentioned principles.

The abdomen slimming apparatus of the present invention comprises a power supply circuit, a timing control circuit, a curing pulse generating circuit, a power amplifying and output circuit, an intensity indicating circuit for the curing pulse signal, and electrode contacts, and is characterized in that said electrode contacts are movable magnetic electrodes positioned in the electrode holes in the bottom of said slimming apparatus, so that said slimming apparatus may be directly placed on the abdomen of the slimmer during operation.

The timing control circuit consists of a programmable oscillator-timer. Each start-up of the timing circuit provides for 10 minutes of curing time, that is, the power source is turned off when the set time has elapsed, and an audio indicator is turned on simultaneously indicating that the curing time of a group of acupuncture points has been completed.

The curing pulse generating circuit comprises: two separated pulse oscillating circuits IC4 sharing a common power supply, for constituting a short acupuncture pulse wave generator of cycle 14.2-18.9 ms, and a long acupuncture pulse wave generator of cycle 260-360 ms, wherein the pulse generated by the short acupuncture pulse wave generator is sent to a pulse output power amplifier via an emitter follower and a differentiating circuit; the pulse generated by the long acupuncture pulse wave generator is sent to a digital counter first, and then sent to the pulse output power amplifier via an emitter follower and an attenuating circuit, and is output from a pulse transformer after being combined with the short acupuncture pulse wave.

The circuitry of the present invention further comprises an acupuncture point group selecting circuit: a three-position interlock switch consisting of a counter, a two-input four-NAND-gate Schmitt trigger and acupuncture point group selecting membrane switches K3-K5 for selecting one out of three curing acupuncture point groups I, II and III, for performing treatment on the corresponding acupuncture point group.

The long acupuncture pulse wave of the present invention occurs no less than 4 pulses each time, while the intermittent time is 1.88-2.16 seconds, the ratio of the amplitudes of the short acupuncture pulse wave to the long acupuncture pulse wave is 1/1.5-1/2.5, and the effective pulse widths of both long acupuncture pulse wave and short acupuncture pulse wave are <1 ms.

A navel positioning aperture or central aperture is provided in the bottom of the abdomen slimming apparatus of the present invention. Taking this navel positioning aperture as the origin of a plane Cartesian coordinate system, the magnetic electrodes are distributed on the horizontal and vertical coordinate axes and in the third and fourth quadrants, and the said magnetic electrodes positioned in the third and fourth quadrants are distributed symmetrically with respect to the vertical axis.

In order to consolidate the slimming effect on the abdomen and to effect slimming of other portions of the human body, the abdomen slimming apparatus of the present invention is provided with an external electrode jack, a pair of external self-bonding electrodes, and an external ear acupuncture point electrode clip.

The movable magnetic electrode of the abdomen slimming apparatus of the present invention may be of two types: one of which implements an electric conductivity effect during treatment by absorbent cotton permeated with physiological saline; and the other implements an electric conductivity effect with self-bonding material.

The abdomen slimming apparatus of the present invention is a combined wave electro-treatment apparatus, the output curing pulse signal has both acupuncture and twirling effects of acupuncture and moxibustion simultaneously, and the speed of acupuncture and moxibustion and the stimulation intensity are much higher than that of common acupuncture and moxibustion. Therefore, the curing effect is high and quick. The magnetic electrodes are placed in the electrode holes in the bottom of the slimming apparatus, which correspond to the abdomen slimming treatment acupuncture points. The abdomen slimming apparatus of the present invention has the following features:
1. Applications: this apparatus has the functions of abdomen slimming and recovery of the abdominal muscles, curing flaccid muscles of the buttocks, waist slimming etc. It can also cure diseases such as habitual constipation;
2. Quick effect: the abdomen line of a pure adiposity person can be reduced by 3-12 cm or more after treatment with this apparatus 1-3 times. A constipation patient can be cured after 1-3 treatments, with consolidated curing effect.
3. Simple and easy to operate: it is easy to use and learn, the user can operate it by oneself.
4. No side-effect: there is no side-effect at all to the human body. A comfortable sense can be felt on the slimming region through the simulation of acupuncture sense by this apparatus during use. It has all the effects of acupuncture, massage, and slimming with exercise, but without the worry of slimming by diet and the side-effects of slimming by fat extraction or medicines.
5. This apparatus is of small size, light weight, and graceful appearance, as well as easy to carry. It can not only be used for home health care, but also as curative apparatus in medical departments, slimming clinics and beauty parlours, as well as ideal health and beauty tools for persons of the literary and art circles.

### The Drawings

Figure 1 is a schematic block diagram of the circuitry of the abdomen slimming apparatus according to the present invention;
Figure 2 is a schematic diagram, divided into parts 2A and 2B, of the circuitry of the abdomen slimming apparatus according to the present invention;
Figure 3 is a front schematic perspective view of the abdomen slimming apparatus according to the present invention;
Figure 4 is a back schematic perspective view of the abdomen slimming apparatus according to the present invention;
Figure 5 is a diagram showing the distribution pattern of the electrode holes in the bottom of the abdomen slimming apparatus according to the present invention; and
Figure 6 is a schematic diagram showing an electrode of the abdomen slimming apparatus according to the present invention.

### Detailed description of the invention

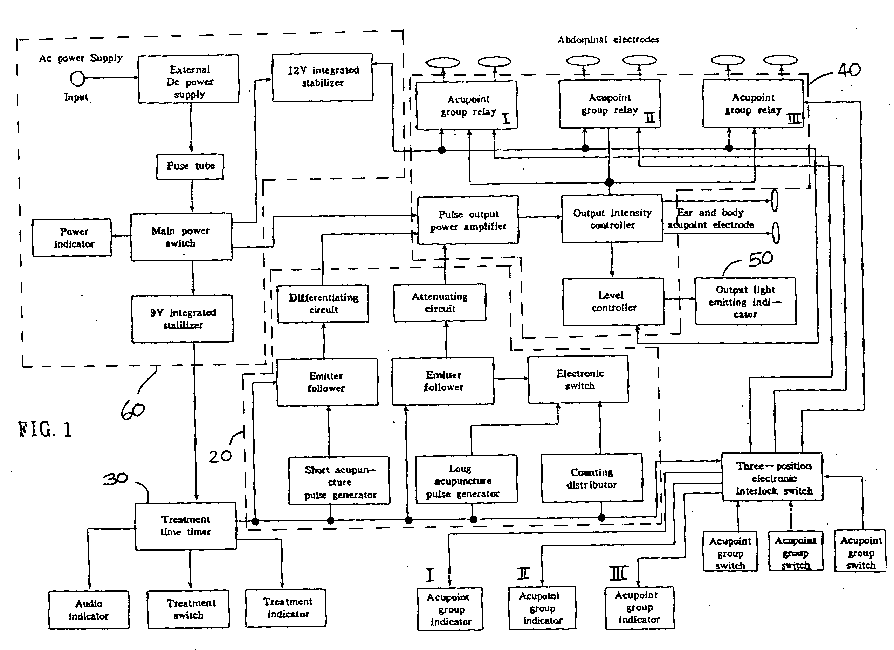

Referring to Figure 1, it is a schematic block diagram of the circuitry of the abdomen slimming apparatus. The circuitry of the present invention mainly comprises a curing pulse generating circuit 20, a timing control circuit 30 for controlling the curing time, a power amplifying and output circuit 40 for amplifying the curing pulse signal, a curing pulse signal intensity indicating circuit 50, and a power supply circuit 60 for providing power to each of the other circuits.

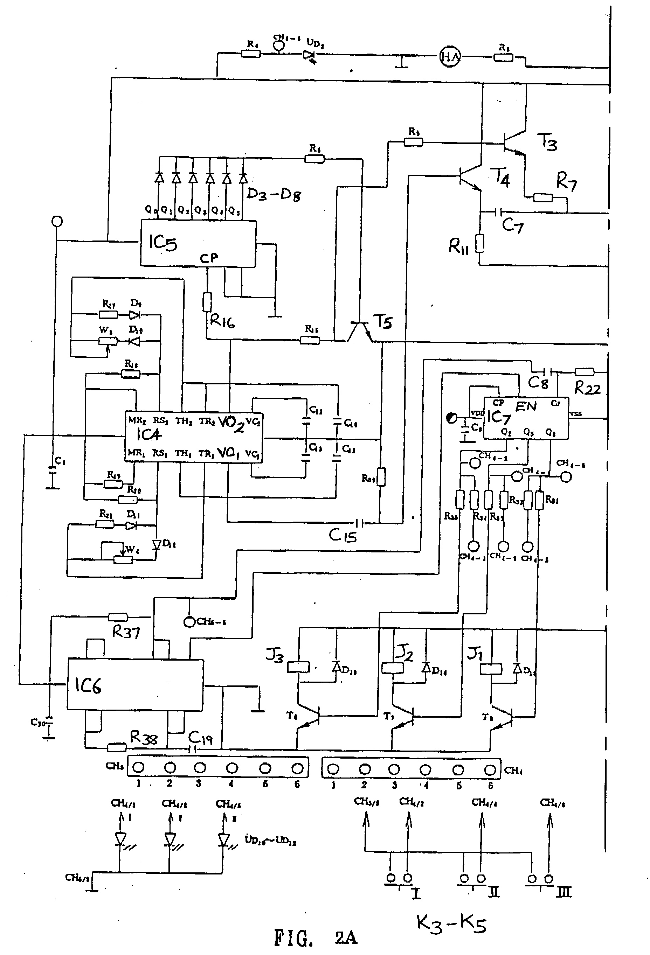

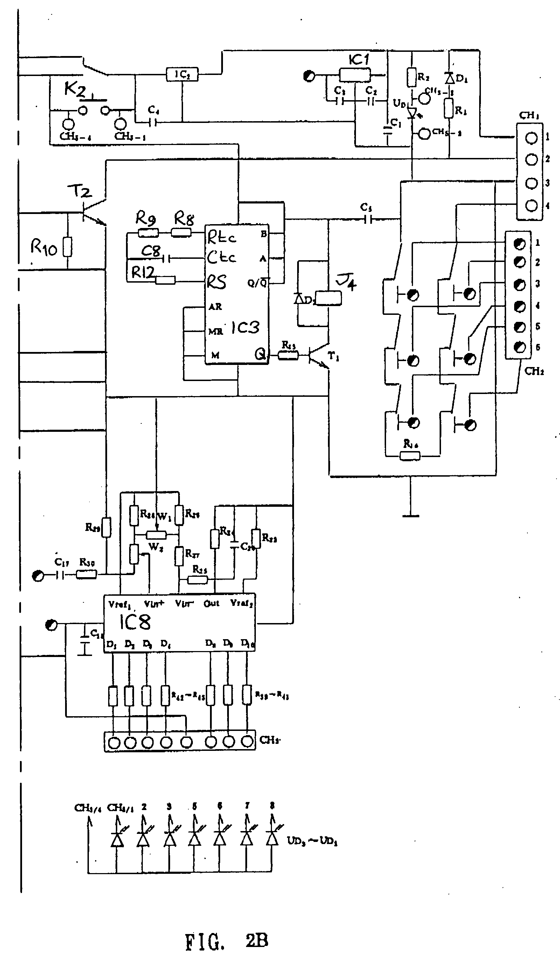

Referring to the circuit diagram of the present invention shown in Figure 2, the following is a detailed explanation of each of the portions:

The timing control circuit of the present invention includes an RC oscillating circuit consisting of a programmable oscillator-timer IC3 and its Rtc and Ctc terminals, resistors R8 and R9, and capacitor C8, the oscillating cycle of which is about 18.7 ms. The timer counts binarily the RS terminal input to IC3 via R12, and output Q is inverted to a low level when a predetermined curing time of 10 minutes is obtained. The relay J4 is then turned off and released, the power source of the main oscillating circuit is opened by the movable normally closed contactor of J4 and the apparatus stops operation. At the same time, the movable normally open contactor turns on the audio indicator, indicating the completion of the curing time of an acupuncture point, and another group of acupuncture points can be selected and cured again by restarting K2.

The curing pulse generating circuit of the present invention includes two separated pulse oscillating circuits IC4 sharing a common power supply to form a short acupuncture pulse generator of cycle 14.2-18.9 ms and a long acupuncture pulse generator of cycle 260-360 ms. These pulses are output by the VO1 terminal and VO2 terminal of IC4, respectively. The terminal VO1 is coupled to the emitter follower composed of T4 and R11 via C15, and then to the base of an NPN power transistor T2 via differentiating circuit C7 and R10. The pulse is output to the short acupuncture pulse wave portion by the pulse transformer B after being amplified.

The terminal VO2 is connected to the terminal CP of the digital counter IC5 via R16 and the terminals Q0-Q5 of IC5 are connected with diodes D3-D8. Diodes D3-D8 are positively turned on by high level output from terminals Q0-Q5 when the pulse signals coming from input CP are counted to Q0-Q5, thereby no signal is output from the collector of T6 when the pulses are counted to Q6-Q9. The base of the emitter follower T3 then acquires four long acupuncture pulse waves output from VO2 of IC4, and these waves are input to the base of T2 after being attenuated by resistor R7. After being amplified, long acupuncture pulse outputs can be obtained owing to the complex application of the counter potential formed by the inductance factor of the pulse transformer 3 and the existing parasitic capacitance.

The apparatus further comprises an acupuncture point group selecting circuit consisting of: a three-position electronic interlock switch consisting of IC6, IC7, and acupuncture point group selecting membrane switches K3-K5, for selecting one out of three curing acupuncture point groups I, II, and III. IC6 is a two-input four-NAND-gate Schmitt trigger, one group of which with resistor R38 and capacitor C19 are used to constitute an inverter oscillating circuit. The reset circuit of IC7 consists of resistors R37, R22 and capacitor C8. The terminal of R37 goes to low level when one of the acupuncture point group selecting membrane switches Kn is turned on, so that the oscillating pulse may enter the EN terminal of IC7 and IC7 begins to count. When it has counted to the Qn correspondingly turned on by the membrane switch Kn, R37 becomes high level, which makes the input terminals of a set of NAND gates in IC6 connected to the oscillating signal become low level through a set of invertors in IC6. Thereby, the thyristor is blocked, IC7 stops counting, and the Qn terminal remains high level. The resistor connected to Qn makes the corresponding transistor conductive, the 12V stabilized voltage supply output by IC1 makes the corresponding relay Jn move into contact and turns on the corresponding group of acupuncture points to perform treatment of the selected group of acupuncture points.

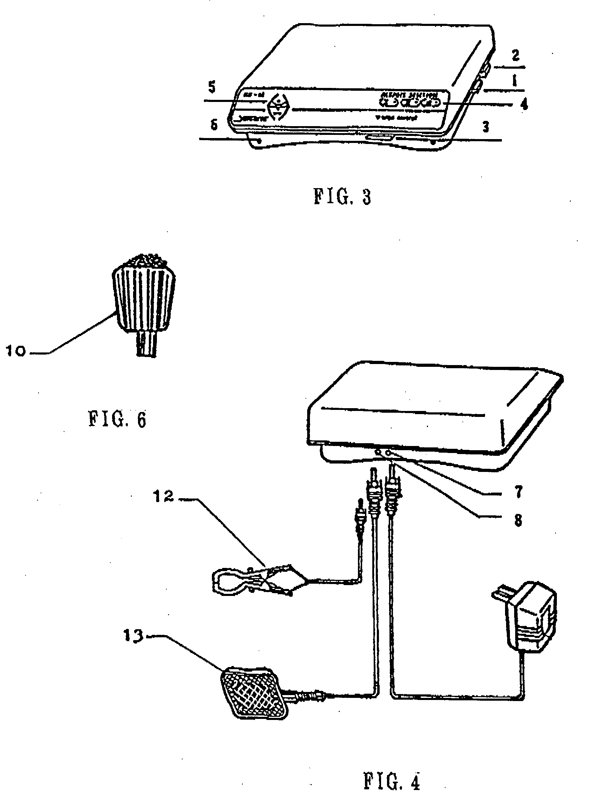

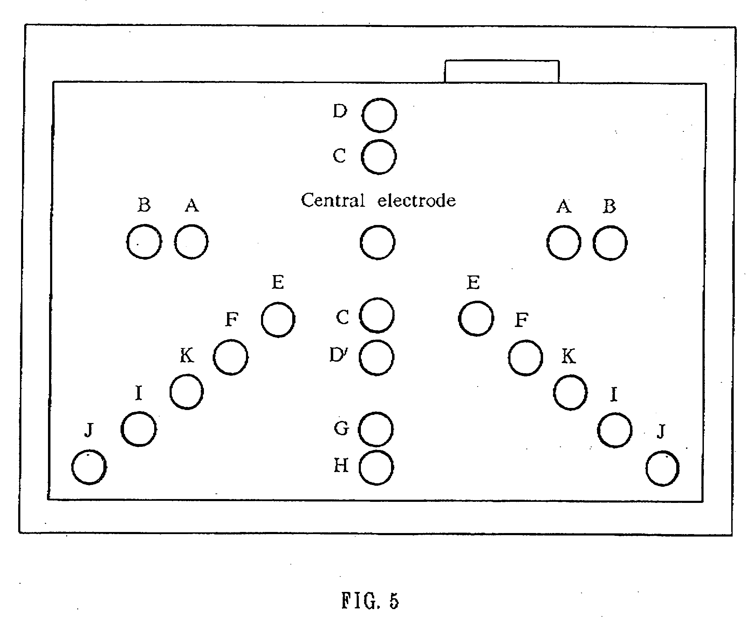

The structure of the abdomen slimming apparatus of the present invention is shown in Figures 3,4,5 and 6. The medical apparatus consists of power supply switch 1, fuse tube 2, output intensity adjuster 3, acupuncture point selecting key 4, treatment key 5, apparatus clamp ring hole 6, DC power supply socket 7, external electrode socket 8, electrode holes (Figure 5) and magnetic electrodes 10. The magnetic electrodes 10 can be inserted into the apparatus during slimming treatment. The ear acupuncture external electrode clip 12 and the external sefl-bonding electrodes 13 are used to consolidate the curing effect of the abdomen slimming treatment or for slimming other portions of human body, for example, the slimming of the buttocks. The insertion positions of the magnetic electrodes 10 of the abdomen slimming apparatus of the present invention are determined depending on the size of waist line as shown in Table 1.

**Table 1**

| Relationship between the size of waistline and the selection of electrode holes | |
|---|---|
| Size of waistline | Symbols of electrode holes selected |
| Less than 90 cm | AA, CC, EE |
| 90-100 cm | AA, DD', FF |
| 100-110 cm | BB, DG, KK |
| 110-120 cm | BB, DC, I I |
| Greater than 120cm | BB, DH, JJ |

How to use the abdomen slimming apparatus of the present invention

### Abdomen slimming:

### 1. Preparations:

A. The user relaxes his/her abdomen, and measures the circumference horizontally at the plumpest portion of the abdomen;
B. The user positions the magnetic electrodes in the corresponding electrode holes of Figure 5 according to Table 1, based on the measurement of the size of the waistline.
C. The user lies down and places the fixing belt under his/her waist, and aligns the central electrode to the navel of the body with the panel of the apparatus oriented towards the head, then inserts the two ends of the fixing belt into the clips on two sides of the apparatus, respectively, and at the same time uniformly pulls the two ends of the belt to fix the belt tightly.

### 2. Slimming Method

A. Connect the power supply, turn on the power supply switch, at this time the apparatus beeps, then the operation can be started.
B. Press the acupuncture point selecting switch 4, the corresponding indicator will light and group I of the acupuncture points are turned on. then press the treatment switch 5 and the light with the symbol"V" will light (the apparatus starts to count time automatically).
C. Slowly adjust the output intensity adjuster 3 from left to right, until the output intensity is adjusted to the maximum endurable intensity of the user.
D. When the prescribed treatment time of group I of the acupuncture points has elapsed, the apparatus stops treatment automatically and beeps at the same time. At that time, the output intensity adjuster should be adjusted to return to its zero reading.
E. The treatment of groups II and III of the acupuncture points is similar to that of group I, except that the respective acupuncture point selecting switch is pressed.
F. One abdomen slimming treatment (about 30 minutes) is completed after the three groups of acupuncture points have been treated in order. Then turn off the power switch 1, loosen the fixing belt and remove the electrodes for future use.

### 3. Period of treatment and consolidation

A. Taking one month as a period of treatment (i.e. one treatment each day stopping treatment for 3 days after every 7 days treatment, so that three cycles take one month), normally the object of slimming can be attained in one period of treatment, depending on the build of the user. Some may require another period of treatment to attain significant slimming effect.
B. In the first 7-day treatment process, the first to the fourth day may only take treatment on the abdomen. In the fifth to the seventh days, group III of the acupuncture points are not treated after the treatment of the groups I and II of acupuncture points of the abdomen, but consolidation treatments are performed by using the external electrodes. The consolidation treatment treats the "Liang' iu (st.34)" and "kungsun (sp.4)" points on the left and right legs, 8 minutes for each leg, and then treats the left and right ears, 10 minutes for each ear, using the ear acupuncture point external electrode clip. The points are taken with reference to the acupuncture point diagram of human body.
C. During the treatment using external electrodes and the ear acupuncture point external electrode clip, insert the electrode plug into the external electrode jack 8 on the back of the apparatus, turn on the power switch 1, press the treatment switch 5, and adjust to the proper intensity to start treatment (independent of the acupuncture point selecting switch).
D. The treatment processes of the second 7 days and the third 7 days are similar to those of the first 7 days. A period of treatment is finished after completion of all the treatments.

### Buttocks slimming

1. Buttocks flaccidness due to excessive fat or lack of exercise can be locally treated employing the external electrode of the apparatus to obtain a healthy and graceful build and remove excessive fat.
2. Acupuncture points: "Huant'iao (G.B.30)," "Ch'engfu (U.B.36)", "Tachui (Du 14)" "Yaoyanykuan (Du 3)" and "Chuliao (femur) (G.B.29)".
3. Treatment time period and sequence of acupuncture points:
   Huant'iao (G.B.30) (10 minutes)
   Ch'eng fu (U.B. 36) (10 minutes)
   Tachui (Du 14) (10 minutes)
   Chuliao (femur) (G.B.29) (10 minutes).
4. The period of treatment is the same as that for abdomen slimming.

### Waist Slimming:

1. Use the external electrode.
2. Acupuncture points: "Shenshu (U.B.23)","Tach'angshu (U.B.25)", "Tachui (Du 14)", "Yaoyangkuan (Du3)", and "Taimai (G.B.26)".
3. Treatment time and sequence of acupuncture points: Shenshu (U.B.23) (10 minutes) Tach'angshu (U.B.25) (10 minutes) Tachui (Du 14) and Yaoyangkuan (Du 3) (10 minutes) Taimai (G.B.26) (10 minutes).
4. The period of treatment is the same as that for abdomen slimming.

### Warnings:

1. Those suffering from cardiac diseases, pacemaker carriers, and pregnant women should not use the invention.
2. Before treatment, the output intensity control key should be set to its zero reading.
3. The abdomen electrode holes should be filled with absorbent cotton, which should be higher than the surface of the electrode and fully permeated with clean water or physiological saline.
4. If aching or scorching of the skin occurs in treatments using the external self-bonding electrodes, the possible causes and appropriate remedies are as follows:
   A. The electrode is not on the proper acupuncture point - reposition it on the proper point;
   B. There is damage to or serious pollution of the electrode surface - wash it or replace it with a new one;
   C. The skin is too dry - damp it slightly with physiological saline;
5. In order to guarantee the curative effect, after each time of use of the external self-bonding electrodes they should be washed with clean water, dried and covered with protective film to be prepared for future use. Note that two self-bonding electrodes should not be bound together to prevent damage to the electrodes and apparatus.
6. When the ear acupuncture point external electrode clip is used, it should be fixed on the ear first before turning on the power (inserting the plug into the socket), and upon completion of treatment, the power should be turned off first before removing the ear acupuncture point external electrode clip from one's ear to avoid short circuit and damage of the elements of the apparatus.

### Experiment 1

1. Persons receiving treatment: 51 corpulent persons in this group, consisting of 13 males and 38 females, 8 unmarried and 8 married below age 25; 13 from age 26 to 35; 9 from age 36 to 45; and 13 over 46 years old.
2. Method of treatment: According to the specification for use of the abdomen slimming apparatus of the present invention, the slimming apparatus was placed on the abdomen with respect to the acupuncture points, each time electro-stimulating the acupuncture points for 10 minutes, i.e. 30 minutes in total for 3 groups of acupuncture points, once every day, for 3 consecutive days.
3. Criteria for evaluation of the curative effect:
A. Reduction of abdomen line of less than 1 cm after 3 times of treatment is regarded as no effect;
B. Reduction of abdomen line of between 1 and 3 cm after 3 times of treatment is regarded as effective;
C. Reduction of abdomen line of between 3 and 10 cm after 3 times of treatment is regarded as significantly effective.

4. The curing effects are shown in Table 2:

**Table 2**

| Reduction of abdomen line after 3 treatments (cm) | Number of persons | Percentage % |
|---|---|---|
| < 1 (no effect) | 11 | 21.6 |
| 1-3 (effective) | 13 | 25.5 |
| > 3 (sig. effective) | 27 | 52.9 |
| Total effective rate | 40 | 78.4 |

### Experiment 2

1. This time, abdomen slimming was practised on 75 corpulent women.
2. The method of treatment and criteria for evaluation of curing effect were the same as those in Experiment 1.
3. The curing effects are shown in Table 3:

**Table 3**

| Reduction cm | Age | | | | total 75 pers. | percentage % |
|---|---|---|---|---|---|---|
| | 20-29 24 pers. | 30-38 18 pers. | 40-49 15 pers. | > 50 18 pers. | | |
| < 1 | 3 | 0 | 0 | 1 | 4 | 5.33 |
| 1-3 | 10 | 7 | 6 | 3 | 26 | 34.67 |
| > 3 | 11 | 11 | 9 | 14 | 45 | 60.00 |
| Total effective rate | 21 | 18 | 16 | 17 | 71 | 94.67 |

It can be seen from the table that the total effective rate in Experiment 2 was 94.67%, and the following features are also revealed by the table: 1. The reduction of abdomen line in the older group is better than than in the younger group. 8 women above 40 years old were observed in this experiment, their reductions of abdomen line were 3-7 cm, and the maximum reduction after 3 times of slimming was 7 cm. 2. The reduction of abdomen line is significant for young women practising slimming postpartum. There were 38 cases in the group of 20-39 years old, most of whose abdomens appeared to be too large. After slimming, their abdomen lines reduced by 2-5 cm;

Therefore, it can be concluded that the effect of the slimming apparatus of the present invention is significant for the elders, for those having a large, soft and slack abdomen, and those having unsatisfactory retraction of the abdomen postpartum.

### Experiment 3

In addition, 66 cases of constipation patients were treated. The cure rate of constipation was 100%. The curing effect is significant, therefore, and the abdomen slimming apparatus of the present invention can also be used to cure constipation.

## Claims

1. Slimming apparatus, comprising a power supply circuit (60), a timing control circuit (30), a pulse generating circuit (20), a power amplifying and output circuit (40), a pulse signal intensity indicating circuit (50), and electrode contacts (10), characterized in that: said electrode contacts are movable magnetic electrodes located in holes in the bottom of said slimming apparatus, which allow said slimming apparatus to be direct directly placed on the abdomen of a slimmer during operation.

2. Apparatus according to claim 1, wherein said timing control circuit (30) consists of a programmable oscillator-timer for measuring a prescribed treatment time, and wherein when the prescribed time has elapsed, the power supply of the output circuit (40) is turned off and an audio indicator is turned on to indicate the completion of the prescribed treatment time.

3. Apparatus according to claim 1 or claim 2, wherein said pulse generating circuit (20) comprises: two separated pulse oscillating circuits IC4 sharing a common power supply, for generating respectively short acupuncture pulse waves of 14.2-18, 9 ms cycle, and long acupuncture pulse waves of 260-360 ms cycle, wherein the short acupuncture pulse waves are sent to a pulse output power amplifier via an emitter follower and a differentiating circuit, and the long acupuncture pulse waves are sent to a digital counter first, then pass through an emitter follower and an attenuating circuit, to the pulse output power amplifier, to combine with the short acupuncture pulse waves, the combined pulse waves being output via a pulse transformer.

4. Apparatus according to claim 3 characterized in that the long acupuncture pulse wave occurs in sequences of no less than 4 pulses with an intermittent time between sequences of 1.68-2.16 seconds.

5. Apparatus according to claim 3 or claim 4, characterized in that the ratio of the amplitude of said short acupuncture pulse waves to said long acupuncture pulse waves is between 1/1.5 and 1/2.5.

6. Apparatus according to any of claims 3, 4 and 5, characterized in that the effective pulse widths of both said long acupuncture pulse waves and short acupuncture pulse waves are less than 1 ms.

7. Apparatus according to any preceding claim, further comprising an acupuncture point group selecting circuit including a three-position electronic interlock switch, which consists of a counter, a two-input, four-NAND-gate Schmitt trigger and acupuncture point group selecting membrane switches K3-K5, for selecting one out of three curing acupuncture point groups I, II and III, to apply treatment to the corresponding point group.

8. Apparatus according to any preceding claim, characterized in that a navel positioning aperture is provided in the bottom of said abdomen slimming apparatus, wherein, taking the navel positioning aperture as the origin of a plane Cartesian coordinate system, said magnetic electrodes are distributed on the horizontal and vertical axes and in the third and fourth quadrants, and said magnetic electrodes in the third and fourth quadrants are symmetrically distributed with respect to the vertical axis.

9. Apparatus according to any preceding claim, further comprising an external electrode jack (8).

10. Apparatus according to any preceding claim, further comprising a pair of external self-bonding electrodes (13).

11. Apparatus according to any preceding claim, further comprising an ear acupuncture point external electrode clip (12).

12. Apparatus according to any preceding claim, characterized in that said movable electrodes are provided with absorbent material permeated with a physiological saline which implements electrical conductivity during treatment.

13. Apparatus according to any of claims 1 to 11, characterized in that said movable electrodes are provided with self-bonding material which implements electrical conductivity during treatment.
